# EUROPEAN PATENT SPECIFICATION

(11) **EP 1 068 898 B1**
(45) Date of publication and mention of the grant of the patent: **29.12.2004**
(21) Application number: 00202435.4
(22) Date of filing: 10.07.2000
(51) Int. Cl.: B01J 29/90, B01J 38/48, B01J 38/50, B01J 38/56, C07C 6/12, C07C 2/86

(54) **Process for the regeneration of zeolitic catalysts used in (trans)alkylation processes**
Verfahren zur Regenerierung von in (Trans-)Alkylierungsverfahren verwendeten Zeolitenkatalysatoren
Procédé de régénération de catalyseurs zéolithiques de (trans)alkylation

(30) Priority: 13.07.1999 IT MI991532
(43) Date of publication of application: 17.01.2001
(73) Proprietor: Polimeri Europa S.p.A., 72100 Brindisi (IT); ENITECNOLOGIE S.p.A., 20097 S. Donato Milanese (Milano) (IT)
(72) Inventor: Amarilli, Stefano, 28014 Maggiora (Novara) (IT); Perego, Carlo, 20040 Carnate (Milano) (IT); Cappellazzo, Oscar, 07041 Alghero (Sassari) (IT); Biddau, Gianfranco, 07046 Porto Torres (Sassari) (IT); Bencini, Elena, 46030 Cerese di Virgilio (Mantova) (IT); Girotti, Gianni, 28100 Novara (IT)
(74) Representative: De Gregori, Antonella

(56) References cited:
- EP-A- 0 847 802
- EP-A- 0 950 650
- US-A- 3 549 557
- US-A- 4 008 291
- US-A- 4 463 209
- US-A- 4 908 341

## Description

The present invention relates to a process for the regeneration of a zeolitic alkylation or transalkylation catalyst, at least partially exhausted, and comprises subjecting said exhausted catalyst to heat treatment with an aromatic hydrocarbon.

More specifically, the process of the present invention is useful for regenerating exhausted zeolitic catalysts which derive from preparation processes of monoalkylated aromatic hydrocarbons by the alkylation of an aromatic hydrocarbon substrate with a suitable alkylating agent selected from C₂-C₄ olefins, isopropanol and mixtures of isopropanol with propylene, or by the transalkylation of an aromatic hydrocarbon substrate with one or more polyalkylaromatic hydrocarbons, said alkylation or transalkylation processes being carried out in at least partially liquid phase and in the presence of a catalyst of zeolitic nature.

The regeneration of these exhausted catalysts according to the present invention comprises treatment with an aromatic hydrocarbon effected in at least partially liquid phase and at a temperature equal to or higher than that at which the alkylation or transalkylation process was carried out.

It has been known for a long time that the alkylation of aromatic hydrocarbon substrates with C₂-C₄ olefins can be effectively carried out by the use of solid catalysts based on zeolites. In 1965 the preparation of cumene by the reaction of benzene with propylene using X or Y zeolite as catalyst, was described for the first time (Minachev, Kr. M., et al, Neftekhimiya 5 (1965) 676). The use of zeolites with a faujasitic structure for the alkylation of benzene with light olefins was subsequently described by Venuto et al. in J. Catal. 5, (1966) 81. U.S. 4,292,458 describes the use of ZSM-5 type zeolites for alkylating benzene with propylene. At present the best results in the alkylation of aromatic substrates with C₂-C₄ olefins, in particular benzene, in at least partially liquid phase, are obtained using beta zeolite as alkylation catalyst (EP 432,814, EP 687,500, EP 847,802).

The alkylation of benzene with isopropanol in the presence of Y zeolite is described in U.S. 5,015,786.

In U.S. 5,160,497, a dealuminated Y zeolite is used, with a molar ratio ranging from 8 to 70, for the alkylation of benzene with propylene and isopropanol.

Preparations of monoalkylated aromatic hydrocarbons by the transalkylation of polyalkylated aromatic hydrocarbons with aromatic substrates in which zeolitic catalysts with small, medium and large pores are used, are described for example in U.S. 3,385,906, U.S. 4,169,111 and EP 308,097.

In particular, the transalkylation reaction of polyalkylated aromatic hydrocarbons can be suitably carried out in a step following the alkylation step of an aromatic hydrocarbon substrate with an olefin, operating on the polyalkylated products which are formed as by-products and are recovered downstream of the alkylation. The use of zeolitic catalysts for preparing monoalkylated aromatic hydrocarbons by the transalkylation of polyalkylated aromatic hydrocarbons in a step subsequent to the alkylation step is described in U.S. 3,385,906, U.S. 4,774,377, U.S. 4,168,111 and EP 308,097, where alkylation and transalkylation processes are combined to obtain better yields to monoalkylated aromatics.

EP 439,632, EP 687,500 and EP 847,802 describe the production of monoalkylated aromatic hydrocarbons from aromatic hydrocarbon substrates, not only by means of alkylation but also by means of transalkylation and a combined alkylation and transalkylation process, catalyzed by beta zeolite. In particular, the alkylation of benzene with ethylene or propylene and the transalkylation of diethylbenzene or diisopropylbenzene with benzene are described.

Zeolites are materials characterized by a lattice structure in which the pore dimensions are of the same order of magnitude as that of the molecules of reagents and reaction products. The high selectivity of these materials in alkylation or transalkylation reactions, for example in the alkylation of benzene to cumene or ethylbenzene, or in the transalkylation of benzene with diethylbenzene or diisopropylbenzene, lies both in its particular acid characteristics and also in the shape-selectivity linked to the specific pore structure of the catalyst.

Owing to the limited channel dimensions in which the acid sites are situated and in which the molecules react, deactivation is a particularly significant phenomenon. It can be caused both by fouling of the single acid sites and also by physical blockage of the channels of the porous system; this is due to the formation of substances with a high molecular weight which obstruct the free passage of reagents and products and hinder the reaction process. The substances responsible for the deactivation of the catalyst are prevalently oligomers, polyalkylated products and polycondensed aromatic rings (anthracenes and higher aromatics) variously substituted. The combination of these substances is hereinafter referred to as pitches.

It is known that, once exhausted, the catalytic material can be partially or totally regenerated by means of thermal treatment at a high temperature (500-600°C) in an oxidative environment (oxygen or air). This in fact allows the combustion of the pitches present in the pores and the regeneration of the material under such conditions as to enable it to be used again in the reaction.

EP 0 353 813 describes the alkylation of aromatic hydrocarbons using alkylating agents containing from 2 to 20 carbon atoms, selected from olefins, alcohols, alkyl halides, esters, ethers and acetylene hydrocarbons. Synthetic or natural materials of the zeolitic or argillaceous type are used as catalysts, which after undergoing deactivation, can be regenerated by means of alternating cyclic washings effected with paraffins and alcohols. The pitches responsible for the deactivation are normally distinguished between soluble and insoluble products of which the latter cause additional weightiness and/or degradation with respect to the former, obstructing access (fully or partially) of the catalyst pores (M. Guisnet et al., in J. Catal. 106, 242-250, 1987 and J. Catal. 134, 286-298, 1992). The effect of the washings carried out in EP 353,813 relates to the dissolution of the polymeric compounds contained in the pitches.

Further examples are EP-A-0950650 disclosing a process for the preparation of 2,6-dimethylnaphthalene in which alkylation on part of the methylating agent of the aromatic hydrocarbon and subsequenty transalkylation of the naphthalene substrate occurs. The zeolite catalyst of the MTW group is reactivated with one or more of the benzene hydrocarbons in at least partially liquid phase, the temperature being at least equal to that used in the preparation process.

US-A-4,008,291 discloses a continuous fixed bed alkylation and reactivation process with a zeolitic catalyst. The reactivation is carried out with an alkylatable hydrocarbon and hydrogen in liquid phase.

US-A-4,908,341 discloses a regeneration process for spent catalyst, containing carbonaceous residue. The spent catalyst is reactivated by contact with aromatic hydrocarbons such as benzene or toluene at temperatures from 600 to 1200°F (315.8-649.4°C).

It has now been surprisingly found that catalysts based on zeolite used in alkylation or transalkylation processes, in particular used in alkylation reactions of aromatic substrates with an alkylating agent selected from C₂-C₄ olefins, isopropanol and mixtures of isopropanol and propylene, or used in transalkylation reactions of aromatic substrates with polyalkylaromatic hydrocarbons, once deactivated due to pitches, can be regenerated by means of heat treatment with an aromatic hydrocarbon.

This treatment not only removes the soluble component of the pitches, but is also capable of chemically degrading, by means of a reactive process, the insoluble pitches: the aromatic hydrocarbon in fact reacts with the pitches, probably by means of a series of alkylation and/or transalkylation reactions catalyzed by the zeolitic material itself, transforming the pitches into molecules characterized by a lower molecular weight, soluble in the aromatic hydrocarbon and, above all, capable of diffusing through the zeolitic pores.

An object of the present invention therefore relates to a process for the regeneration of a zeolitic catalyst at least partially exhausted, deriving from a preparation process of a monoalkylated aromatic compound by means of the alkylation of an aromatic hydrocarbon substrate with an alkylating agent selected from olefins containing from two to four carbon atoms, isopropanol and mixtures of isopropanol and propylene, or by means of the transalkylation of an aromatic hydrocarbon substrate selected among benzene, toluene or their mixtures, with one or more polyalkylaromatic hydrocarbons, effected in the presence of the zeolitic catalyst and in at least partially liquid phase, said regeneration process comprising subjecting said exhausted catalyst to treatment with an aromatic hydrocarbon alone in liquid phase and at a temperature at least equal to that at which the alkylation or transalkylation process from which the exhausted catalyst derives, was carried out, said regeneration temperature being comprised between 200 and 290°C.

The aromatic hydrocarbon used for the regenerating treatment of the exhausted catalyst can be selected from benzene, toluene, naphthalene and their mixtures.

A preferred aspect of the present invention is that the aromatic hydrocarbon used for the regenerating treatment is equal to the aromatic substrate of the alkylation process from which the exhausted catalyst derives, or is equal to the aromatic substrate of the transalkylation process from which the catalyst to be regenerated derives.

The treatment is carried out for a time ranging from 0.1 to 60 hours, preferably from 15 to 45 hours, and at a pressure ranging from 20 to 40 bars.

The regeneration process is preferably carried out at a temperature which is higher than that of the alkylation or transalkylation process from which the exhausted catalyst derives.

Exhausted zeolitic catalysts which can be subjected to regeneration treatment according to the present invention are those containing a zeolite selected from beta zeolite, Y zeolite, mordenite, ZSM-12, MCM-22, and are preferably those containing beta zeolite.

The alkylation process, from which these exhausted catalysts derive, can be all the known alkylation processes in which an aromatic hydrocarbon substrate is alkylated with an alkylating agent selected from a C₂-C₄ olefin, isopropanol and mixtures of isopropanol and propylene, in the presence of a zeolitic catalyst and in at least partially liquid phase. The aromatic substrate which is alkylated can be benzene, toluene, naphthalene or their mixtures, and is preferably benzene; the olefin can be ethylene or propylene.

In particular, exhausted catalysts containing Y zeolite deriving from the alkylation process of benzene with ethylene or propylene to give cumene or ethylbenzene described in U.S. 5,177,285, EP 521,554 and EP 485,683, exhausted catalysts containing MCM-22 zeolite deriving from the alkylation of benzene with C₂-C₄ olefins described in U.S. 4,992,606, exhausted catalysts containing ZSM-12 zeolite or mordenite deriving from the alkylation of benzene with C₂-C₄ olefins as described in Rao B. S., et al. "Proceedings, 1^{st} Tokyo Conference on Advanced Catalyst Science and Technology", Tokyo, July 1-5, 1990 (S. Yoshida et al., Eds), Vol. 1, 56, page 361, can be regenerated according to the method of the present invention. Y zeolite is described in U.S. 3,130,007, MCM-22 zeolite is described in EP 64,205.

Exhausted catalysts containing Y zeolite deriving from the alkylation process of benzene with propanol to give cumene described in U.S. 5,015,786 and exhausted catalysts containing dealuminated Y zeolite used in the alkylation of benzene with isopropanol and propylene described in U.S. 5,160,497, can be regenerated according to the method of the present invention.

According to a preferred aspect, catalysts containing beta zeolite used for the alkylation of benzene with ethylene to give ethylbenzene or for the alkylation of benzene with propylene to give cumene described in EP 432,814, EP 439,632, EP 629,599, EP 687,500 and EP 847,802, can be regenerated according to the method of the present invention. In particular, the catalytic compositions comprising beta zeolite described in EP 687,500 and EP 847,802 are catalytic compositions consisting of beta zeolite and an inorganic ligand characterized by an extra-zeolite porosity consisting for a fraction of at least 25% of pores with a radius higher than 100 Å and, in the case of EP 847,802 also characterized by a total extra-zeolitic pore volume greater than or equal to 0.80 ml/g.

According to another preferred aspect, exhausted zeolitic catalysts can be regenerated according to the method of the present invention, deriving from a process for the alkylation of aromatic compounds by reaction of the aromatic compound of interest with isopropanol, alone or mixed with an olefin, consisting in carrying out said alkylation reaction in the presence of a catalytic composition based on zeolite, under mixed gas-liquid phase conditions or under completely liquid phase conditions at such temperature and pressures that the concentration of water in the liquid reaction phase is not higher than 8,000 ppm, regardless of the total content of water present in the reaction mixture. In this alkylation process, the aromatic compound is preferably benzene and the olefin, when present, is propylene, the zeolite is beta zeolite, preferably in acid form, described in U.S. 3,308,069. This beta zeolite can be mixed with suitable binding agents, such as for example, alumina, or catalytic compositions can be used, comprising beta zeolite described in EP 687,500 and EP 847,802, i.e. catalytic compositions consisting of beta zeolite and an inorganic ligand characterized by an extra-zeolite porosity consisting, for a fraction of at least 25%, of pores with a radius higher than 100 Å and, in the case of EP 847,802 also characterized by a total extra-zeolitic pore volume greater than or equal to 0.80 ml/g. This alkylation process is preferably carried out at a temperature ranging from 170 to 230°C, and at a reaction pressure ranging from 1 to 50 bars under mixed phase conditions using isopropanol and mixtures of isopropanol and propylene indifferently as alkylating agent. Experts in the field are able to define a priori - starting from the molar ratio benzene/isopropanol and propylene/isopropanol when propylene is also fed to the reaction section together with isopropylic alcohol - the temperature and pressure conditions which ensure that the concentration limit of water present in the liquid phase equal to about 8,000 ppm, regardless of the total quantity of water present in the reaction mixture and therefore regardless of the quantity of isopropanol fed to the reaction section, is respected.

The transalkylation processes from which exhausted catalysts which can be regenerated with the process of the present invention, derive, are all known transalkylation processes in which an aromatic substrate is transalkylated with one or more polyalkylaromatic hydrocarbons in the presence of a zeolitic catalyst and in at least partially liquid phase. The aromatic substrate is benzene, toluene, naphthalene or their mixtures, and is preferably benzene, the polyalkylaromatic hydrocarbon can be selected from diethylbenzene, optionally mixed with triethylbenzene, and diisopropylbenzene, optionally mixed with triisopropylbenzene.

In particular, exhausted catalysts containing Y zeolite deriving from the transalkylation process of benzene with diethylbenzene or diisopropylbenzene described for example in I.I. Lishchiner et al., React. Kinet. Catal. Lett., Vol. 23, Nrs. 3-4, 261-265 (1983), exhausted catalysts containing MCM-22 zeolite used in the transalkylation of polyalkylaromatics, such as for example the transalkylation of diisopropylbenzene described in U.S. 5,371,310, exhausted catalysts containing ZSM-12 zeolite or mordenite used in the transalkylation of polyalkylaromatics described in EP 308,097, can be regenerated according to the method of the present invention.

According to a preferred aspect, catalysts containing beta zeolite used for the transalkylation of diethylbenzene with benzene and diisopropylbenzene with benzene described in EP 432,814, EP 439,632, EP 629,599, EP 687,500 and EP 847,802, can be regenerated according to the method of the present invention.

The process of the present invention can be effected by recovering the exhausted catalyst from the alkylation or transalkylation reactor and subjecting it to regenerating treatment with the pre-selected aromatic hydrocarbon in a specific reactor, but a preferred aspect of the present invention is that the regeneration process of the catalyst, at least partially exhausted, is effected in the same alkylation or transalkylation reactor, by feeding the aromatic hydrocarbon selected for the regenerating treatment, after suspending the feeding of the alkylation or transalkylation reagents. This embodiment is particularly convenient and preferred when the alkylation or transalkylation reactor is a continuous reactor, even more preferably a catalytic fixed bed continuous reactor.

When the regeneration process of the at least partially exhausted catalyst is carried out in the same alkylation or transalkylation reactor, a particularly preferred aspect of the present invention is to use, as aromatic hydrocarbon for the regenerating treatment, the same aromatic substrate used in the alkylation or transalkylation process, after suspending the feeding of the alkylating reagent or polyalkylbenzene, respectively, to the reactor. This embodiment is particularly convenient and preferred when the alkylation or transalkylation reactor is a continuous reactor, even more preferably a catalytic fixed bed continuous reactor. In practice, according to this particular embodiment in continuous of the regeneration process of the present invention, when a zeolitic alkylation catalyst is exhausted, the feeding stream of the alkylating agent to the alkylation reactor is suspended, said alkylating agent being selected from C₂-C₄ olefins, isopropanol or mixtures of isopropanol and propylene, whereas the stream of aromatic substrate continues to be fed to the reactor, optionally raising the temperature of the catalytic bed, when it has been decided to carry out the regeneration treatment at a higher temperature than that of the alkylation process. When the regeneration process is concluded, the feeding of the alkylating agent is restarted to re-begin the alkylation process, after possible cooling of the catalytic bed. This particular embodiment is very useful in the case of the regeneration of an exhausted catalyst containing beta zeolite, Y zeolite or MCM-22 zeolite, preferably beta zeolite, deriving from the alkylation of benzene with ethylene to give ethylbenzene or of benzene with propylene to give cumene, or deriving from the alkylation of benzene with isopropanol, alone or mixed with propylene.

According to a particular embodiment in continuous of the regeneration process of the present invention, when an exhausted catalyst deriving from a transalkylation process is to be regenerated, the feeding polyalkylaromatic hydrocarbon stream to the transalkylation reactor is suspended, whereas the stream of aromatic substrate continues to be fed to the reactor, optionally raising the temperature of the catalytic bed when it has been decided to carry out the regeneration treatment at a higher temperature than that of the transalkylation process. When the regeneration process is concluded, the feeding of the polyalkylated aromatic is restarted to re-begin the transalkylation process, after possible cooling of the catalytic bed. This particular embodiment is very useful in the case of the regeneration of an exhausted catalyst containing beta zeolite, Y zeolite or MCM-22 zeolite, preferably beta zeolite, deriving from the transalkylation of benzene with diethylbenzene, optionally mixed with triethylbenzene, or from the transalkylation of benzene with diisopropylbenzene, optionally mixed with triisopropylbenzene.

In all cases in which the regeneration is carried out in the same alkylation or transalkylation reactor, and said reactor is in continuous, a WHSV preferably ranging from 1 to 20 h⁻¹ is adopted, even more preferably from 2 to 8 h⁻¹.

In accordance with what is specified above, a further aspect of the present invention relates to an alkylation process in continuous, in the presence of a zeolitic catalyst, preferably in a fixed bed reactor, of an aromatic substrate with an alkylating agent selected from olefins containing from 2 to 4 carbon atoms, isopropanol and mixtures of isopropanol and propylene, with cyclic regeneration of the catalyst, comprising the following steps:
a) alkylation of a feeding of aromatic substrate with a feeding of alkylating agent, carried out in at least partially liquid phase and in the presence of a zeolitic catalyst, until the catalyst shows at least partial deactivation;
(b) suspension of the feeding of the alkylating agent and treatment of said deactivated catalyst with the aromatic substrate alone, in at least partially liquid phase and at a temperature at least equal to the alkylation temperature of step a), until the catalyst has been regenerated;
c) re-establishment of the temperature conditions used in the alkylation in step a) when the regeneration has been carried out at a temperature higher than that of the alkylation step, and re-starting of the feeding of the alkylating agent.

The regeneration process of step b) is preferably carried out at a WHSV ranging from 1 to 20 hours⁻¹, preferably from 2 to 8 hours⁻¹. The temperature of this step preferably ranges from 200 to 290°C.

The aromatic substrate is preferably benzene and, when the alkylating agent is an olefin, said olefin is preferably selected from ethylene and propylene. The zeolitic catalyst preferably contains a zeolite selected from beta zeolite and Y zeolite, and is preferably beta zeolite. The reaction conditions of step a) are those described in literature and known to experts in the field or those described above relating to the alkylation of aromatic substrates, in the presence of beta zeolite, with isopropanol, alone or mixed with propylene.

In accordance with what is specified above, a further aspect of the present invention relates to a transalkylation process in continuous, in the presence of a zeolitic catalyst, preferably in a fixed bed reactor, of an aromatic substrate with one or more polyalkylaromatics, with cyclic regeneration of the catalyst, comprising the following steps:
a) transalkylation of a feeding of aromatic substrate with a feeding of polyalkylaromatic hydrocarbon, carried out in at least partially liquid phase and in the presence of a zeolitic catalyst, until the catalyst shows at least partial deactivation;
(b) suspension of the feeding of the polyalkylaromatic and treatment of said deactivated catalyst with the aromatic substrate alone, in at least partially liquid phase and at a temperature at least equal to the transalkylation temperature of step a), until the catalyst has been regenerated;
c) re-establishment of the temperature conditions used in the transalkylation in step a) when the regeneration has been carried out at a temperature higher than that of the transalkylation step, and re-starting of the feeding of the polyalkylaromatic.

The regeneration process of step b) is preferably carried out at a WHSV ranging from 1 to 20 h⁻¹, preferably from 2 to 8 h⁻¹. The temperature of this step preferably ranges from 200 to 290°C.

The aromatic substrate is preferably benzene and the aromatic hydrocarbon is preferably selected from diethylbenzene, optionally mixed with triethylbenzene, and diisopropylbenzene, optionally mixed with triisopropylbenzene. The zeolitic catalyst preferably contains a zeolite selected from beta zeolite and Y zeolite, and is preferably beta zeolite. The process conditions of step (a) are those described in the prior art and are known to experts in the field.

The regeneration of exhausted catalysts according to the present invention and in accordance with what is described above, is conveniently used also in the case of catalysts deriving from a preparation process of a monoalkylated aromatic hydrocarbon comprising the following steps:
1) alkylation of a feeding of aromatic substrate with a feeding of an alkylating agent selected from C₂-C₄ olefins, isopropanol and mixtures of isopropanol and propylene, carried out in at least partially liquid phase and in the presence of a zeolitic catalyst;
2) separation of the product obtained in (a) into a fraction containing the aromatic substrate (b), a fraction containing a monoalkylated aromatic hydrocarbon and (c) a fraction containing polyalkylated aromatic hydrocarbons,
3) transalkylation of the fraction containing polyalkylated aromatic hydrocarbons with the aromatic substrate, carried out in at least partially liquid phase and in the presence of a zeolitic catalyst.

In this multi-stepped process the regeneration of the exhausted catalysts can be carried out by contemporaneously suspending the feeding of both the alkylating reagent and polyalkylaromatic hydrocarbon, and letting the aromatic substrate flow alone until the catalysts have been regenerated, or, preferably, the regeneration of the catalyst of step (a) and the catalyst of step (b) are carried out independently.

### EXAMPLE 1

An alkylation test of benzene with propylene to give cumene is carried out using an experimental apparatus consisting of an AISI316 fixed bed micro-pilot reactor, with an internal diameter of 1 cm., a total length of 8.5 cm, tanks for the benzene and propylene in the feeding, dosage pumps for the feeding of the two reagents in liquid phase, an automatic system for the temperature and pressure control, for discharging the effluent from the reactor and for analysis of the feed and product. The catalyst used in this test is a catalyst based on beta zeolite similar to that prepared and described in example 4 of patent application EP 0 847 802. 4.5 g of said catalyst, sieved into granules having dimensions equal to 20-40 mesh, and 11.5 g of inert material, are charged into the reactor, obtaining a homogeneous catalytic bed having a height equal to 8.5 cm. Benzene and propylene are fed, at a temperature of 150°C, a pressure of 30 barg, a WHSV of 19.6 g/g/hour and a benzene/propylene ratio of 6 m/m, obtaining a weight percentage of cumene in the effluent of 19.6. The test is prolonged until the concentration of cumene in the effluent drops to below 5% by weight (3.72%). At this point the regeneration procedure of the exhausted catalyst is initiated:
- the feeding of propylene is interrupted whereas the feeding of benzene remains unaltered (WHSV = 19.6 g/g/hour);
- the catalytic bed is heated to a temperature of 220°C (at a rate of 0.6°C/min);
- the above temperature is maintained for 3 hours;
- the catalytic bed is cooled to a temperature of 150°C.

At the end of the regeneration procedure described above, the feeding of propylene is re-started, the alkylation process is re-activated as described above, and a weight percentage of cumene is obtained in the effluent equal to 19.51. The test is prolonged until the concentration of cumene in the effluent drops to 6.20% by weight. At this point a regeneration procedure of the exhausted catalyst analogous to that described above, is initiated. At the end of this second regeneration, the alkylation process is re-started, obtaining a weight percentage of cumene in the effluent equal to 19.65.

### EXAMPLE 2

An alkylation test of benzene with propylene to give cumene is carried out using an experimental apparatus consisting of an AISI316 fixed bed micro-pilot reactor, with an internal diameter of 1 cm., a total length of 8.5 cm, tanks for the benzene and propylene in the feeding, dosage pumps for the feeding of the two reagents in liquid phase, an automatic system for the temperature and pressure control, for discharging the effluent from the reactor and for the analysis of the feed and product. The catalyst used in this test is a catalyst based on beta zeolite similar to that prepared and described in example 4 of patent application EP 0 847 802. 4.5g of said catalyst, sieved into granules having dimensions equal to 20-40 mesh, and 11.5 g of inert material, are charged into the reactor, obtaining a homogeneous catalytic bed having a height equal to 8.5 cm. Benzene and propylene are fed, at a temperature of 150°C, a pressure of 30 barg, a WHSV of 19.6 g/g/hour and a benzene/propylene ratio of 6 m/m, obtaining a weight percentage of cumene in the effluent of 19.44. The test is prolonged until the concentration of cumene in the effluent drops to below 5% by weight (3.04%). At this point the regeneration procedure of the exhausted catalyst is initiated:
- the feeding of propylene is interrupted
- the flow-rate of the benzene feeding is reduced to a value of WHSV = 4.0 g/g/hour
- the catalytic bed is heated to a temperature of 220°C (at a rate of 0.6°C/min);
- the above temperature is maintained for 24 hours;
- the catalytic bed is cooled to a temperature of 150°C.

At the end of the regeneration procedure described above, the original flow-rate of benzene is re-established and also the feeding of propylene, the alkylation process is re-activated as described above, and a weight percentage of cumene is obtained in the effluent equal to 19.31. The test is prolonged until the concentration of cumene in the effluent drops to 4.42% by weight. At this point a regeneration procedure of the exhausted catalyst analogous to that described above, is initiated. At the end of this second regeneration, the alkylation process is re-started, obtaining a weight percentage of cumene in the effluent equal to 19.28.

### EXAMPLE 3

An alkylation test of benzene with propylene to give cumene is carried out using an experimental apparatus consisting of an AISI316 fixed bed micro-pilot reactor, with an internal diameter of 1.2 cm., a total length of 22 cm, tanks for the benzene and propylene in the feeding, dosage pumps for the feeding of the two reagents in liquid phase, an automatic system for the temperature and pressure control, for discharging the effluent from the reactor and for the analysis of the feed and product.

The catalyst used in this test is a commercial Y zeolite (called 330 HUA, of Tosoh Corporation).

The conditions for the alkylation reaction are the following:

| | |
|---|---|
| Temperature at the inlet = | 150°C |
| Pressure = | 38 barg |
| WHSV = | 20 g/g/h |
| [Benzene]/[Propylene] = | 7 m/m |

1.2 g of catalyst (obtained first by forming tablets of catalyst in powder form and then grinding and sieving into granules having dimensions equal to 20-40 mesh) and 30 g of inert material are charged into the reactor, obtaining a homogeneous catalytic bed having a height equal to 22 cm. The feeding of the reagents is started, obtaining a weight percentage of cumene in the effluent equal to 13.71.

When the concentration of cumene in the effluent drops to below 5% by weight (2.9%), the following regeneration procedure is initiated:
- the feeding of propylene is interrupted
- the catalytic bed is heated to a temperature of 200°C;
- the above temperature is maintained for 5 hours;
- the catalytic bed is cooled to a temperature of 150°C.

At the end of the regeneration procedure described above, the feeding of propylene is re-started, the alkylation process is re-activated as described above, and a weight percentage of cumene is obtained in the effluent equal to 13.61. When the concentration of cumene in the effluent drops to below 5% by weight (2.1%), a regeneration procedure analogous to that described above, is initiated. At the end of the regeneration, the alkylation process is re-started, obtaining a weight percentage of cumene in the effluent equal to 13.55.

### EXAMPLE 4 (comparative)

An alkylation test of benzene with propylene to give cumene is carried out using a catalyst based on beta zeolite and the experimental apparatus described in example 1.

The reaction conditions during the test are the following:

| | |
|---|---|
| Temperature at the inlet = | 150°C |
| Pressure = | 30 barg |
| WHSV = | 20.0 g/g/h |
| [Benzene]/[Propylene] = | 6 m/m |

4.5 g of the catalyst of example 1, ground and sieved into granules having dimensions equal to 20-40 mesh, and 11.5 g of inert material are charged into the reactor, obtaining a homogeneous catalytic bed having a height equal to 8.5 cm. The reagents are fed and a weight percentage of cumene is obtained in the effluent equal to 19.7.

The test is prolonged and when the concentration of cumene in the effluent drops to below 5% (1.01%) the following regeneration procedure of the exhausted catalyst is initiated:
- the feeding of propylene and benzene is interrupted
- the system is flushed with nitrogen
- n-hexane is fed with the same flow-rate used for the benzene in the alkylation phase
- the catalytic bed is heated to a temperature of 200°C (at a rate of 0.6°C/min);
- the above temperature is maintained for 3 hours;
- the catalytic bed is cooled to a temperature of 150°C.

At the end of the regeneration procedure described above, the system is flushed again with nitrogen, the feeding of benzene and propylene and the alkylation test are re-started as described above, obtaining a weight percentage of cumene in the effluent of 1.66. A second regeneration procedure is effected, in the same way as described before, using cyclohexane instead of n-hexane. At the end of the regeneration procedure described, the system is flushed again with nitrogen and the feeding of benzene and propylene and alkylation test are re-started as described above, obtaining a weight percentage of cumene in the effluent of 0.93.

## Claims

1. A process for the regeneration of a zeolitic catalyst at least partially exhausted, deriving from a preparation process of a monoalkylated aromatic compound by means of the alkylation of an aromatic hydrocarbon substrate with an alkylating agent selected from olefins containing from two to four carbon atoms, isopropanol and mixtures of isopropanol and propylene, or by means of the transalkylation of an aromatic substrate selected among benzene, toluene or their mixtures, with one or more polyalkylated aromatic hydrocarbons, effected in the presence of a zeolitic catalyst and in at least partially liquid phase, said regeneration process comprising subjecting the exhausted catalyst to treatment with an aromatic hydrocarbon alone in liquid phase and at a temperature equal or higher than that at which the alkylation or transalkylation process, from which the exhausted catalyst derives, was carried out, said regeneration temperature being comprised between 200 and 290°C.

2. The process according to claim 1, wherein the aromatic hydrocarbon used for the regeneration treatment is selected from benzene, toluene, naphthalene and their mixtures.

3. The process according to claim 1, carried out a time ranging from 0.1 to 60 hours.

4. The process according to claim 3, carried out for a time ranging from 15 to 35 hours.

5. The process according to claim 1, carried out at a pressure ranging from 20 to 40 bars.

6. The process according to claim 1, wherein the zeolitic catalyst is a catalyst containing a zeolite selected from beta zeolite, Y zeolite, mordenite, ZSM-12, MCM-22.

7. The process according to claim 5, wherein the zeolite is beta zeolite.

8. The process according to one of the claims from 1 to 7, wherein the exhausted catalyst derives from an alkylation process of an aromatic substrate with an alkylating agent selected from olefins containing from two to four carbon atoms, isopropanol and mixtures of isopropanol and propylene, carried out in at least partially liquid phase.

9. The process according to claim 8, wherein the aromatic substrate is benzene, toluene, naphthalene or their mixtures.

10. The process according to claim 9, wherein the aromatic substrate is benzene.

11. The process according to claim 8, wherein the alkylating agent is ethylene or propylene.

12. The process according to claim 8, wherein the exhausted catalyst is based on beta zeolite and derives from an alkylation process of benzene with an alkylating agent selected from ethylene, propylene, isopropanol and mixtures of isopropanol with propylene.

13. The process according to claim 7, wherein the aromatic hydrocarbon used for the regenerating process is equal to the aromatic substrate of the alkylation process.

14. The process according to claim 7, carried out in the same alkylation reaction.

15. The process according to claim 14, wherein the reactor is a continuous reactor.

16. The process according to claim 15, wherein the reactor is a fixed bed continuous reactor.

17. The process according to claim 13 and 16, wherein the regeneration treatment is carried out in continuous using as aromatic hydrocarbon the same aromatic substrate stream as the alkylation process.

18. The process according to claim 17, wherein an exhausted catalyst based on beta zeolite deriving from an alkylation process of benzene with an alkylating agent selected from ethylene, propylene, isopropanol and mixtures of isopropanol with propylene, is regenerated in the same alkylation reactor using the benzene stream.

19. The process according to one of the claims from 1 to 7, wherein the exhausted catalyst derives from a transalkylation process of an aromatic hydrocarbon selected among benzene, toluene or their mixtures substrate with a polyalkylated aromatic hydrocarbon, carried out in at least partially liquid phase.

20. The process according to claim 19, wherein the aromatic substrate is benzene.

21. The process according to claim 19, wherein the polyalkylated aromatic hydrocarbon is diisopropylbenzene, optionally mixed with triisopropylbenzene, or diethylbenzene, optionally mixed with triethylbenzene.

22. The process according to claim 19, wherein the exhausted catalyst is based on beta zeolite and derives from a transalkylation process of benzene with diisopropylbenzene, optionally mixed with triisopropylbenzene, or diethylbenzene, optionally mixed with triethylbenzene.

23. The process according to claim 19, wherein the aromatic hydrocarbon used for the regenerating process is equal to the aromatic substrate of the transalkylation process.

24. The process according to claim 19, carried out in the same alkylation reactor.

25. The process according to claim 24, wherein the reactor is a continuous reactor.

26. The process according to claim 25, wherein the reactor is a fixed bed continuous reactor.

27. The process according to claim 23 and 26, wherein the regeneration treatment is carried out in continuous using as aromatic hydrocarbon the same aromatic substrate stream as the transalkylation process.

28. The process according to claim 27, wherein an exhausted catalyst based on beta zeolite deriving from a transalkylation process of benzene with diisopropylbenzene, optionally mixed with triisopropylbenzene, or diethylbenzene, optionally mixed with triethylbenzene, is regenerated in the same transalkylation reactor using the benzene stream.

29. An alkylation process in a continuous reactor of an aromatic substrate with an alkylating agent selected from olefins containing from 2 to 4 carbon atoms, isopropanol and mixtures of isopropanol with propylene, in the presence of a zeolitic catalyst, with cyclic regeneration of the zeolitic catalyst, comprising the following steps:
a) alkylation of a feeding of aromatic substrate with a feeding of alkylating agent, carried out in at least partially liquid phase and in the presence of the zeolitic catalyst, until the catalyst shows at least partial deactivation;
(b) suspension of the feeding of the alkylating agent and treatment of said deactivated catalyst with the feeding of the aromatic substrate alone, in at least partially liquid phase and at a temperature at least equal to the alkylation temperature of step a) said renegeration temperature being comprised between 200 and 290° until the catalyst has been at least partially regenerated;
c) re-establishment of the temperature conditions used in the alkylation in step a) when the regeneration has been carried out at a temperature higher than that of the alkylation step, and re-starting of the feeding of the alkylating agent.

30. The process according to claim 29, wherein the deactivated catalyst is based on beta zeolite, the aromatic substrate is benzene and the alkylating agent is selected from ethylene, propylene, isopropanol, mixtures of isopropanol with propylene.

31. A transalkylation process in a continuous reactor of an aromatic substrate with a polyalkylated aromatic hydrocarbon, in the presence of a zeolitic catalyst, with cyclic regeneration of the zeolitic catalyst, comprising the following steps:
a) transalkylation of a feeding of aromatic substrate selected among benzene, toluene or their mixtures with a feeding of polyalkylated aromatic hydrocarbon, carried out in at least partially liquid phase and in the presence of a zeolitic catalyst, until the catalyst shows at least partial deactivation;
(b) suspension of the feeding of the polyalkylaromatic hydrocarbon and treatment of said deactivated catalyst with the feeding of the aromatic substrate alone, in liquid phase and at a temperature at least equal to the transalkylation temperature of step a), said regeneration temperature being comprised between 200 and 290°C, until the catalyst has been at least partially regenerated;
c) re-establishment of the temperature conditions used in the transalkylation in step a) when the regeneration has been carried out at a temperature higher than that of the transalkylation step, and re-starting of the feeding of the polyalkylated aromatic hydrocarbon.

32. The process according to claim 31, wherein the deactivated catalyst is based on beta zeolite, the aromatic substrate is benzene and the polyalkylated aromatic hydrocarbon is selected from diisopropylbenzene, optionally mixed with triisopropylbenzene, and diethylbenzene, optionally mixed with triethylbenzene.

33. The process according to claim 17, 29 or 31, wherein the regeneration is carried out at a WHSV ranging from 1 to 20 h⁻¹.

34. The process according to claim 33, wherein the regeneration is carried out at a WHSV ranging from 2 to 8.

35. The regeneration process according to claim 1, wherein the temperature at which the regeneration of the exhausted catalyst is carried out is higher than the temperature of the alkylation or transalkylation process from which the exhausted catalyst derives.

## Patentansprüche

1. Verfahren zur Regenerierung eines zumindest teilweise erschöpften Zeolithkatalysators aus einem Herstellungsprozeß einer monoalkylierten aromatischen Verbindung mittels Alkylierung eines aromatischen Kohlenwasserstoffsubstrats mit einem aus Olefinen mit zwei bis vier Kohlenstoffatomen, Isopropanol und Gemischen von Isopropanol und Propylen ausgewählten Alkylierungsmittel oder mittels Transalkylierung eines unter Benzol, Toluol oder deren Gemischen ausgewählten aromatischen Substrats mit einem oder mehreren polyalkylierten aromatischen Kohlenwasserstoffen, durchgeführt in Gegenwart eines Zeolithkatalysators und in zumindest teilweise Flüssigphase, wobei das Regenerierungsverfahren darin besteht, daß der erschöpfte Katalysator einer Behandlung mit einem aromatischen Kohlenwasserstoff allein in Flüssigphase und bei einer Temperatur gleich der oder höher als diejenige, bei der der Alkylierungs- oder Transalkylierungsprozeß durchgeführt wurde, aus dem der erschöpfte Katalysator stammt, unterzogen wird, und die Regenerierungstemperatur zwischen 200 und 290ºC liegt.

2. Verfahren nach Anspruch 1, bei dem der für die Regenerierungsbehandlung verwendete aromatische Kohlenwasserstoff aus Benzol, Toluol, Naphthalen und deren Gemischen ausgewählt wird.

3. Verfahren nach Anspruch 1, durchgeführt über einen Zeitraum von 0,1 bis 60 Stunden.

4. Verfahren nach Anspruch 3, durchgeführt über einen Zeitraum von 15 bis 35 Stunden.

5. Verfahren nach Anspruch 1, durchgeführt bei einem Druck von 20 bis 40 bars.

6. Verfahren nach Anspruch 1, bei dem der Zeolithkatalysator ein Katalysator ist, der einen aus Beta-Zeolith, Y-Zeolith, Mordenit, ZSM-12, MCM-22 ausgewählten Zeolith enthält.

7. Verfahren nach Anspruch 5, bei dem der Zeolith Beta-Zeolith ist.

8. Verfahren nach einem der Ansprüche 1 bis 7, bei dem der erschöpfte Katalysator aus einem Alkylierungsprozeß eines aromatischen Substrats mit einem aus zwei bis vier Kohlenstoffatome enthaltenden Olefinen, Isopropanol und Mischungen von Isopropanol und Propylen ausgewählten Alkylierungsmittel stammt, durchgeführt in zumindest teilweise Flüssigphase.

9. Verfahren nach Anspruch 8, bei dem das aromatische Substrat Benzol, Toluol, Naphthalen oder deren Gemische ist.

10. Verfahren nach Anspruch 9, bei dem das aromatische Substrat Benzol ist.

11. Verfahren nach Anspruch 8, bei dem das Alkylierungsmittel Ethylen oder Propylen ist.

12. Verfahren nach Anspruch 8, bei dem der erschöpfte Katalysator auf Beta-Zeolith basiert und aus einem Alkylierungsprozeß von Benzol mit einem aus Ethylen, Propylen, Isopropanol und Gemischen von Isopropanol mit Propylen ausgewählten Alkylierungsmittel stammt.

13. Verfahren nach Anspruch 7, bei dem der für das Regenerierungsverfahren verwendete aromatische Kohlenwasserstoff gleich dem aromatischen Substrat des Alkylierungsprozesses ist.

14. Verfahren nach Anspruch 7, durchgeführt in der gleichen Alkylierungsreaktion.

15. Verfahren nach Anspruch 14, bei dem der Reaktionsapparat ein Durchlauf-Reaktionsapparat ist.

16. Verfahren nach Anspruch 15, bei dem der Reaktionsapparat ein Durchlauf-Reaktionsapparat mit ortsfestem Bett ist.

17. Verfahren nach Anspruch 13 und 16, bei dem die Regenerierungsbehandlung kontinuierlich unter Verwendung des gleichen aromatischen Substratstroms wie der Alkylierungsprozeß als aromatischer Kohlenwasserstoff durchgeführt wird.

18. Verfahren nach Anspruch 17, bei dem ein auf Beta-Zeolith basierender erschöpfter Katalysator aus einem Alkylierungsprozeß von Benzol mit einem aus Ethylen, Propylen, Isopropanol und Gemischen von Isopropanol mit Propylen ausgewählten Alkylierungsmittel in dem gleichen Alkylierungs-Reaktionsapparat unter Verwendung des Benzolstroms regeneriert wird.

19. Verfahren nach einem der Ansprüche 1 bis 7, bei dem der erschöpfte Katalysator aus einem Transalkylierungsprozeß eines unter Benzol, Toluol oder deren Gemischen ausgewählten aromatischen Kohlenwasserstoffsubstrats mit einem polyalkylierten aromatischen Kohlenwasserstoff stammt, durchgeführt in zumindest teilweise Flüssigphase.

20. Verfahren nach Anspruch 19, bei dem das aromatische Substrat Benzol ist.

21. Verfahren nach Anspruch 19, bei dem der polyalkylierte aromatische Kohlenwasserstoff Diisopropylbenzol, wahlweise gemischt mit Triisopropylbenzol, oder Diethylbenzol, wahlweise gemischt mit Triethylbenzol, ist.

22. Verfahren nach Anspruch 19, bei dem der erschöpfte Katalysator auf Beta-Zeolith basiert und aus einem Transalkylierungsprozeß von Benzol mit Diisopropylbenzol, wahlweise gemischt mit Triisopropylbenzol, oder Diethylbenzol, wahlweise gemischt mit Triethylbenzol, stammt.

23. Verfahren nach Anspruch 19, bei dem der für das Regenerierungsverfahren verwendete aromatische Kohlenwasserstoff gleich dem aromatischen Substrat des Transalkylierungsprozesses ist.

24. Verfahren nach Anspruch 19, durchgeführt in dem gleichen Alkylierungs-Reaktionsapparat.

25. Verfahren nach Anspruch 24, bei dem der Reaktionsapparat ein Durchlauf-Reaktionsapparat ist.

26. Verfahren nach Anspruch 25, bei dem der Reaktionsapparat ein Durchlauf-Reaktionsapparat mit ortsfestem Bett ist.

27. Verfahren nach Anspruch 23 und 26, bei dem die Regenerierungsbehandlung kontinuierlich unter Verwendung des gleichen aromatischen Substratstroms wie der Transalkylierungsprozeß als aromatischer Kohlenwasserstoff durchgeführt wird.

28. Verfahren nach Anspruch 27, bei dem ein auf Beta-Zeolith basierender erschöpfter Katalysator aus einem Transalkylierungsprozeß von Benzol mit Diisopropylbenzol, wahlweise gemischt mit Triisopropylbenzol, oder Diethylbenzol, wahlweise gemischt mit Triethylbenzol, in dem gleichen Transalkylierungs-Reaktionsapparat unter Verwendung des Benzolstroms regeneriert wird.

29. Alkylierungsverfahren in einem Durchlauf-Reaktionsapparat eines aromatischen Substrats mit einem Alkylierungsmittel, ausgewählt aus Olefinen mit zwei bis vier Kohlenstoffatomen, Isopropanol und Gemischen von isopropanol mit Propylen, in Gegenwart eines Zeolithkatalysators, bei zyklischer Regenerierung des Zeolithkatalysators, mit den folgenden Schritten:
a) Alkylierung einer Zuführung eines aromatischen Substrats mit einer Zuführung eines Alkylierungsmittels, durchgeführt in zumindest teilweise Flüssigphase und in Gegenwart des Zeolithkatalysators, bis der Katalysator eine zumindest teilweise Entaktivierung zeigt;
b) Aussetzung der Zuführung des Alkylierungsmittels und Behandlung des entaktivierten Katalysators mit der Zuführung des aromatischen Substrats allein in Flüssigphase und bei einer Temperatur zumindest gleich der Alkylierungstemperatur des Schrittes a), wobei die Regenerierungstemperatur zwischen 200 und 290ºC liegt, bis der Katalysator zumindest teilweise regeneriert ist;
c) Wiederherstellung der bei der Alkylierung im Schritt a) verwendeten Temperaturbedingungen, wenn die Regenerierung bei einer höheren Temperatur als derjenigen des Alkylierungsschrittes durchgeführt wurde, und erneutes Starten der Zuführung des Alkylierungsmittels.

30. Verfahren nach Anspruch 29, bei dem der entaktivierte Katalysator auf Beta-Zeolith basiert, das aromatische Substrat Benzol ist und das Alkylierungsmittel aus Ethylen, Propylen, Isopropanol und Gemischen von Isopropanol mit Propylen ausgewählt ist.

31. Transalkylierungsverfahren in einem Durchlauf-Reaktionsapparat eines aromatischen Substrats mit einem polyalkylierten aromatischen Kohlenwasserstoff in Gegenwart eines Zeolithkatalysators bei zyklischer Regenerierung des Zeolithkatalysators, mit den folgenden Schritten:
a) Transalkylierung einer Zuführung eines unter Benzol, Toluol oder deren Gemischen ausgewählten aromatischen Substrats mit einer Zuführung von polyalkyliertem aromatischem Kohlenwasserstoff, ausgeführt in zumindest teilweise Flüssigphase und in Gegenwart eines Zeolithkatalysators, bis der Katalysator eine zumindest teilweise Entaktivierung zeigt;
b) Aussetzung der Zuführung des polyalkylierten aromatischen Kohlenwasserstoffs und Behandlung des entaktivierten Katalysators mit der Zuführung des aromatischen Substrats allein in Flüssigphase und bei einer Temperatur zumindest gleich der Transalkylierungstemperatur des Schrittes a), wobei die Regenerierungstemperatur zwischen 200 und 290ºC liegt, bis der Katalysator zumindest teilweise regeneriert worden ist;
c) Wiederherstellen der bei der Transalkylierung im Schritt a) verwendeten Temperaturbedingungen, wenn die Regenerierung bei einer höheren Temperatur als derjenigen des Transalkylierungsschrittes durchgeführt wurde, und erneutes Starten der Zuführung des polyalkylierten aromatischen Kohlenwasserstoffs.

32. Verfahren nach Anspruch 31, bei dem der entaktivierte Katalysator auf Beta-Zeolith basiert, das aromatische Substrat Benzol ist und der polyalkylierte aromatische Kohlenwasserstoff aus Diisopropylbenzol, wahlweise gemischt mit Triisopropylbenzol, und Diethylbenzol, wahlweise gemischt mit Triethylbenzol, ausgewählt ist.

33. Verfahren nach Anspruch 17, 29 oder 31, bei dem die Regenerierung mit einer Raumgeschwindigkeit (WHSV) im Bereich von 1 bis 20 h⁻¹ durchgeführt wird.

34. Verfahren nach Anspruch 33, bei dem die Regenerierung mit einer Raumgeschwindigkeit (WHSV) im Bereich von 2 bis 8 h⁻¹ durchgeführt wird.

35. Regenerierungsverfahren nach Anspruch 1, bei dem die Temperatur, bei der die Regenerierung des erschöpften Katalysators durchgeführt wird, höher ist als die Temperatur des Alkylierungs- oder Transalkylierungsprozesses, aus dem der erschöpfte Katalysator stammt.

## Revendications

1. Procédé pour la régénération d'un catalyseur zéolitique au moins partiellement épuisé, dérivant d'un procédé de préparation d'un composé aromatique monoalkylé au moyen de l'alkylation d'un substrat hydrocarbure aromatique avec un agent d'alkylation choisi parmi des oléfines contenant de deux à quatre atomes de carbone, de l'isopropanol et des mélanges d'isopropanol et de propylène, ou au moyen de la transalkylation d'un substrat aromatique choisi parmi le benzène, le toluène ou leurs mélanges, avec un ou plusieurs hydrocarbures aromatiques polyalkylés, effectuée en présence d'un catalyseur zéolitique et en phase au moins partiellement liquide, ledit procédé de régénération comprenant la soumission du catalyseur épuisé à un traitement avec un hydrocarbure aromatique seul, en phase liquide et à une température égale ou supérieure à celle à laquelle le procédé d'alkylation ou de transalkylation, duquel le catalyseur épuisé dérive, est conduit, ladite température de régénération étant comprise entre 200 et 290°C.

2. Procédé selon la revendication 1, dans lequel l'hydrocarbure aromatique utilisé pour le traitement de régénération est choisi parmi le benzène, le toluène, le naphtalène et leurs mélanges.

3. Procédé selon la revendication 1, conduit durant un temps allant de 0,1 à 60 heures.

4. Procédé selon la revendication 3, conduit durant un temps allant de 15 à 35 heures.

5. Procédé selon la revendication 1, conduit à une pression allant de 20 à 40 bars.

6. Procédé selon la revendication 1, dans lequel le catalyseur zéolitique est un catalyseur contenant une zéolite choisie parmi la zéolite bêta, la zéolite Y, la mordénite, ZSM-12, MCM-22.

7. Procédé selon la revendication 5, dans lequel la zéolite est la zéolite bêta.

8. Procédé selon l'une des revendications 1 à 7, dans lequel le catalyseur épuisé dérive d'un procédé d'alkylation d'un substrat aromatique avec un agent d'alkylation choisi parmi des oléfines contenant de deux à quatre atomes de carbone, de l'isopropanol et des mélanges d'isopropanol et de propylène, conduit en phase au moins partiellement liquide.

9. Procédé selon la revendication 8, dans lequel le substrat aromatique est le benzène, le toluène, le naphtalène et leurs mélanges.

10. Procédé selon la revendication 9, dans lequel le substrat aromatique est le benzène.

11. Procédé selon la revendication 8, dans lequel l'agent d'alkylation est l'éthylène ou le propylène.

12. Procédé selon la revendication 8, dans lequel le catalyseur épuisé est à base de zéolite bêta et dérive d'un procédé d'alkylation de benzène avec un agent d'alkylation choisi parmi l'éthylène, le propylène, l'isopropanol et des mélanges d'isopropanol et de propylène.

13. Procédé selon la revendication 7, dans lequel l'hydrocarbure aromatique utilisé pour le procédé de régénération est égal au substrat aromatique du procédé d'alkylation.

14. Procédé selon la revendication 7, conduit dans la même réaction d'alkylation.

15. Procédé selon la revendication 14, dans lequel le réacteur est un réacteur continu.

16. Procédé selon la revendication 15, dans lequel le réacteur est un réacteur continu à lit fixe.

17. Procédé selon les revendications 13 et 16, dans lequel le traitement de régénération est conduit en continu en utilisant en tant qu'hydrocarbure aromatique le même courant de substrat aromatique que le procédé d'alkylation.

18. Procédé selon la revendication 17, dans lequel un catalyseur épuisé à base de zéolite bêta dérivant d'un procédé d'alkylation de benzène avec un agent d'alkylation choisi parmi l'éthylène, le propylène, l'isopropanol et des mélanges d'isopropanol et de propylène, est régénéré dans le même réacteur d'alkylation en utilisant le courant de benzène.

19. Procédé selon l'une des revendications 1 à 7, dans lequel le catalyseur épuisé dérive d'un procédé de transalkylation d'un substrat hydrocarbure aromatique choisi parmi le benzène, le toluène ou leurs mélanges avec un hydrocarbure aromatique polyalkylé, conduit en phase au moins partiellement liquide.

20. Procédé selon la revendication 19, dans lequel le substrat aromatique est le benzène.

21. Procédé selon la revendication 19, dans lequel l'hydrocarbure aromatique polyalkylé est le diisopropylbenzène, facultativement mélangé avec le triisopropylbenzène ou le diéthylbenzène, facultativement mélangé avec le triéthylbenzène.

22. Procédé selon la revendication 19, dans lequel le catalyseur épuisé est à base de zéolite bêta et dérive d'un procédé de transalkylation de benzène avec le diisopropylbenzène, facultativement mélangé avec le triisopropylbenzène ou le diéthylbenzène, facultativement mélangé avec le triéthylbenzène.

23. procédé selon la revendication 19, dans lequel l'hydrocarbure aromatique utilisé pour le procédé de régénération est égal au substrat aromatique du procédé de transalkylation.

24. procédé selon la revendication 19, conduit dans le même réacteur d'alkylation.

25. Procédé selon la revendication 24, dans lequel le réacteur est un réacteur continu.

26. Procédé selon la revendication 25, dans lequel le réacteur est un réacteur continu à lit fixe.

27. Procédé selon les revendications 23 et 26, dans lequel le traitement de régénération est conduit en continu en utilisant en tant qu'hydrocarbure aromatique le même courant de substrat aromatique que le procédé de transalkylation.

28. Procédé selon la revendication 27, dans lequel un catalyseur épuisé à base de zéolite bêta dérivant d'un procédé de transalkylation de benzène avec le diisopropylbenzène, facultativement mélangé avec le triisopropylbenzène ou le diéthylbenzène, facultativement mélangé avec le triéthylbenzène, est régénéré dans le même réacteur de transalkylation en utilisant le courant de benzène.

29. Procédé d'alkylation dans un réacteur continu d'un substrat aromatique avec un agent d'alkylation choisi parmi des oléfines contenant de 2 à 4 atomes de carbone, de l'isopropanol et des mélanges d'isopropanol et de propylène, en présence d'un catalyseur zéolitique, avec régénération cyclique du catalyseur zéolitique, comprenant les étapes suivantes :
a) alkylation d'une charge de substrat aromatique avec une charge d'agent d'alkylation, conduite en phase au moins partiellement liquide et en présence du catalyseur zéolitique, jusqu'à ce que le catalyseur présente une désactivation au moins partielle ;
b) suspension de la charge d'agent d'alkylation et traitement dudit catalyseur désactivé avec la charge de substrat aromatique seule, en phase liquide et à une température au moins égale à la température d'alkylation de l'étape a), ladite température de régénération étant comprise entre 200 et 290°C, jusqu'à ce que le catalyseur ait été au moins partiellement régénéré ;
c) rétablissement des conditions de température utilisées dans l'alkylation dans l'étape a) lorsque la régénération a été conduite à une température supérieure à celle de l'étape d'alkylation, et redémarrage de la charge de l'agent d'alkylation.

30. Procédé selon la revendication 29, dans lequel le catalyseur désactivé est à base de zéolite bêta, le substrat aromatique est le benzène et l'agent d'alkylation est choisi parmi l'éthylène, le propylène, l'isopropanol, des mélanges d'isopropanol et de propylène.

31. Procédé de transalkylation dans un réacteur continu d'un substrat aromatique avec un hydrocarbure aromatique polyalkylé, en présence d'un catalyseur zéolitique, avec régénération cyclique du catalyseur zéolitique, comprenant les étapes suivantes :
a) transalkylation d'une charge de substrat aromatique choisi parmi le benzène, le toluène ou leurs mélanges avec une charge d'hydrocarbures aromatiques polyalkylés, conduite en phase au moins partiellement liquide et en présence d'un catalyseur zéolitique, jusqu'à ce que le catalyseur présente une désactivation au moins partielle ;
b) suspension de la charge d'hydrocarbure polyalkylaromatique et traitement dudit catalyseur désactivé avec la charge de substrat aromatique seule, en phase liquide et à une température au moins égale à la température de transalkylation de l'étape a), ladite température de régénération étant comprise entre 200 et 290°C, jusqu'à ce que le catalyseur ait été au moins partiellement régénéré ;
c) rétablissement des conditions de température utilisées dans la transalkylation dans l'étape a) lorsque la régénération a été conduite à une température supérieure à celle de l'étape de transalkylation, et redémarrage de la charge d'hydrocarbure aromatique polyalkylé.

32. Procédé selon la revendication 31, dans lequel le catalyseur désactivé est à base de zéolite bêta, le substrat aromatique est le benzène et l'hydrocarbure aromatique polyalkylé est choisi parmi le diisopropylbenzène, facultativement mélangé avec le triisopropylbenzène ou le diéthylbenzène, facultativement mélangé avec le triéthylbenzène.

33. Procédé selon la revendication 17, 29 ou 31, dans lequel la régénération est conduite à un WHSV allant de 1 à 20 h⁻¹.

34. Procédé selon la revendication 33, dans lequel la régénération est conduite à un WHSV allant de 2 à 8 h⁻¹.

35. Procédé de régénération selon la revendication 1, dans lequel la température à laquelle la régénération du catalyseur épuisé est conduite est supérieure à la température du procédé d'alkylation ou de transalkylation duquel le catalyseur épuisé dérive.
